(19) Europäisches Patentamt
European Patent Office
Office européen des brevets

(11) EP 1 493 452 B1

# (12) EUROPEAN PATENT SPECIFICATION

(45) Date of publication and mention of the grant of the patent:
**03.02.2010 Bulletin 2010/05**

(51) Int Cl.:
***A61L 15/46** (2006.01)* ***A61L 15/26** (2006.01)*
***A61L 15/42** (2006.01)* ***A61L 15/58** (2006.01)*

(21) Application number: **04076789.9**

(22) Date of filing: **18.06.2004**

## (54) Antimicrobially active flexible hydrophilic polyurethane foam

Antimikrobiell wirksamer flexibeler hydrophiler Polyurethanschaumstoff

Mousse de polyuréthane hydrophile flexible ayant une activité antimicrobiènne

(84) Designated Contracting States:
**AT BE BG CH CY CZ DE DK EE ES FI FR GB GR HU IE IT LI LU MC NL PL PT RO SE SI SK TR**

(30) Priority: **18.06.2003 EP 03076909**

(43) Date of publication of application:
**05.01.2005 Bulletin 2005/01**

(73) Proprietor: **Corpura B.V.**
**4041 CL Kesteren (NL)**

(72) Inventor: **Verweire, Ineke**
**9112 Sinaai-Waas (BE)**

(74) Representative: **Van Reet, Joseph et al**
**Gevers & Vander Haeghen,**
**Holidaystraat 5**
**1831 Diegem (BE)**

(56) References cited:
**EP-A- 0 190 814**
**EP-A- 0 235 949**
**WO-A-02/062403**
**GB-A- 2 170 713**
**US-A- 4 631 297**
**US-A- 5 302 385**
**US-A1- 2002 177 828**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 493 452 B1

## Description

The invention relates to an antimicrobially active flexible hydrophilic material, which is in particular suited for use in body care applications, such as wound care, incontinence care, ostomy care, skin care and cosmetic care, and which comprises a hydrophilic polyurethane foam. The invention more particularly relates to wound dressings and particularly but not exclusively to such dressings that are useful in the treatment of heavily exuding wounds.

In advanced wound care, it is believed that wet wound care has significant advantages. An ideal wound dressing should have the following characteristics:

1) remove excess exudate from the wound surface;
2) maintain high humidity at the wound dressing interface to promote healing;
3) provide thermal insulation and cushioning of the wound;
4) allow gas exchanges and the passage of water vapour;
5) not leave dressing residues as polymeric particles or fibres, nor leach out toxic substances;
6) be impermeable to micro-organisms;
7) not adhere to new tissue formed in the wound; and
8) not cause pain nor trauma during the exchange of the dressing

A lot of dressings which try to fulfil all these requirements are already commercially available. Different materials such as hydrocolloids, alginates, hydrogels and hydrophilic foams, membranes and barrier films, or combination of these materials are described in the prior art. An example of a wound dressing comprising a hydrophilic polyurethane foam is described in US-A-2003/0088202. Although hydrophilic polyurethane foams are quite suitable for wet wound care, they have the disadvantage that hydrophilic polyurethane foam dressings as such do not affect possible present infections or bacterial contaminations which are likely to occur during dressing exchange. Deep and open wounds are extremely susceptible to infection, because of the lack of a skin barrier and the excess of food the wound offers to bacteria. Moreover the risk to be infected with bacteria which are resistant to antibiotics has increased significantly in the hospital environment. Infected wounds heal very difficult and are very difficult to treat.

In common, infected wounds are locally treated with antimicrobially active agents, applied directly in a pure, dissolved or emulsified form to the wound and mostly covered with a gauze or advanced dressing. Examples of such antimicrobial agents are mercury agents such as thimerosal (Merthiolate®) or merbromin (Mercurochrome ®) or silver compounds as silver salts (f.i. silvernitrate 0.5-1 % solutions), silver sulfadiazine complex (f.i. trade names Silvadene® and Flammazine®, or iodine tincture ( as Isobetadine®).

However, applying these agents directly to the wound shows certain disadvantages:

- they may cause cytotoxic reactions, due to the toxicity of the agent or the compound components;
- they can attack and destroy cells and substances in the wound fluid that promote wound healing, whereby the wound healing process is slowed down or even inhibited;
- high concentrations are needed causing acquired resistance and/or allergic reactions;
- they are often unstable in light
- they are difficult to handle; and
- they need to be applied several times a day.

In order to avoid these negative effects, different dressings have been developed.

In WO 99/13923 (Tyco group) for example, a hydrogel wound dressing is described, which may comprise a bacteriostatic and/or antimicrobial agent, particularly bismuth tribromophenate or particularly silversulfadiazine. The bacteriostatic and/or antimicrobial agent is released in the wound.

In various medical devices (f.i. Actisorb® of J&J, Avance® of SSL International) antiseptics based on the silver ion are used, the antibacterial activity of the silver compound being combined with the active wound exudate management of the dressing. In WO 02/062403 (Coloplast), a medical dressing is described which consists of a hydrophilic polyurethane foam. In order to make this foam antimicrobially effective, an antimicrobial silver complex, which is capable of releasing the silver ion into the wound, is incorporated in the foam.

A drawback of dressings having a releasable antimicrobial agent is that they may have adverse effects on the wound healing process, because of toxic reactions to healthy cells or influencing the activity of healing body agents or inflammation reactions as a result of eliminating the antimicrobial agent residues. Moreover there is a need to renew periodically the dressing to maintain its antimicrobial properties.

The object of the present invention is to provide a new hydrophilic polyurethane foam material which is rendered antimicrobially active without having to incorporate therein an antimicrobially active compound which has to be released.

To this end, the material according to the present invention is characterised in that said material comprises

further at least one antimicrobially active quaternary ammonium compound which has an apolar end group and which is covalently bonded to the hydrophilic polyurethane foam in such an amount that the material is antimicrobially active and has:

- a fluid absorption capacity after saturation of the material of higher than 4 g per gram material, preferably higher than 8 g, more preferably higher than 12 g, and most preferably higher than 15 g per gram material;
- a fluid absorption capacity after draining of the material of higher than 2 g, preferably higher than 6 g, more preferably higher than 10 g, and most preferably higher than 13 g per gram material; and
- a wet out, lower than 500 s, preferably lower than 250 s, more preferably lower than 50 s and most preferably lower than 10 s.

Due to the fact the antimicrobially active quaternary ammonium compound is covalently bonded to the hydrophilic polyurethane foam, it will not leach, especially not into the wound when used as wound dressing, so that the material according to the present invention does not show the problems of the prior art dressings containing leachable antimicrobial agents.

A polyurethane foam comprising an antimicrobially active quaternary ammonium compound which has an apolar end group and which is covalently bonded to the foam to render this foam antimicrobially active, is already disclosed in US-A-4 631 297 (Dow Corning). Because of the use of toxic leachable catalysts and surfactants, the polyurethane foams disclosed in this US patent are however not suitable for medical use. Moreover, they are not hydrophilic. From the examples given in this US patent, it appears that a relatively large amount of the quaternary ammonium compound is required to render the polyurethane foams antimicrobially effective. To achieve an effective antimicrobial activity, 3 and 5 parts of the ammonium compound had to be added to 100 parts of the polyol, corresponding to about 2 and 3% on the total foam.

Based on the teachings of US-A-4 631 297 it is not obvious that a hydrophilic polyurethane foam can be made antimicrobially effective by means of the antimicrobially active quaternary ammonium compounds without loosing the desired hydrophilic properties of the polyurethane foam. Due to their apolar end group, these quaternary ammonium compounds have indeed a considerable hydrophobing effect on the foam, in particular when used in the amounts which are required to be effective according to US-A-4 631 297. This effect is mentioned for example in US-A-2002/0177828. In most of the examples of this US patent application, use is made of antimicrobially active quaternary ammonium compounds which do not have apolar end groups, in order to obtain the desired hydrophilic properties. These quaternary ammonium compounds are covalently bonded to a gauze-type substrate and to one another to form a polymeric superabsorbent surface on the substrate. In this way, the treated substrates had an increased water absorption capacity and were antimicrobial effective. A drawback of the materials disclosed in US-A-2002/0177828 is however that a quite large amount of the antimicrobially active quaternary ammonium compounds is required and that the achieved superabsorbant polymer surface may become a slippery gel material after water absorption. In US-A-2002/0177828 tests have also been done with an antimicrobially active quaternary ammonium compound having an apolar end group, more particularly with TMS (3-(trimethoxysilyl)-propyloctadecyldimethyl ammonium chloride). During the testing there were however difficulties due to the hydrophobic or water-repellent nature of the siloxane treated material (cotton gauze) and, notwithstanding the relatively high TMS content (8 and 9 %), the treated gauze did not show significant antibacterial activity.

In US-A-5 045 322 the antimicrobially active quaternary ammonium compounds disclosed in US-A-4 631 297 were used in superabsorbent polymer gels to give them antimicrobial properties. In the examples different amounts of TMS were covalently bonded to an acrylic acid-based polymer gel. Antimicrobial tests showed that quite large amounts of TMS were required (11 and 20%) to achieve an antimicrobial activity.

In view of the above described prior art, the present inventor has found quite surprisingly that the antimicrobially active quaternary ammonium compounds which have an apolar end group are so effective in hydrophilic polyurethane foams, that the amount thereof, which is required to achieve the desired antimicrobial activity of the foam, is so small that the desired fluid absorption properties can be maintained.

In a preferred embodiment of the material according to the invention, the quaternary ammonium compound is covalently bonded to the hydrophilic polyurethane foam in an amount of less than 1 part by weight, preferably less than 0.80 parts by weight, more preferably less than 0.60 parts by weight, and most preferably less than 0.40 parts by weight per 100 parts by dry weight of said material. As already mentioned, it is quite surprising that such small amounts of the quaternary ammonium compound, which enable to maintain the fluid absorption characteristics of the material, are sufficient in a hydrophilic polyurethane foam to achieve the desired antimicrobial properties. When comparing these amounts with the amounts required in US-A-4 631 297 and in US-A-5 045 322, it can be seen that smaller amounts of the quaternary ammonium compound are required to make a hydrophilic polyurethane foam antimicrobial compared to a hydrophobic polyurethane foam or a superabsorbent acrylate or methacrylate polymer.

Other particularities and advantages of the invention will become apparent from the following description of some particular embodiments of the antimicrobially active hydrophilic material according to the present invention.

The present invention relates to antimicrobially active flexible hydrophilic materials which comprise a hydrophilic polyurethane foam. These materials are in particular suitable for use in body care applications, such as wound care, incontinence care, ostomy care, skin care and cosmetic care. The present invention in particular relates to wound dressings comprising such a material according to the invention. These wound dressings may be self-adhering or non-adhering.

The antimicrobially active flexible material according to the invention is self-adhering or non-adhering and comprises a polyurethane foam having covalently bonded thereto at least one antimicrobially active quaternary ammonium compound having an apolar end group. The polyurethane foam substrate is hydrophilic and able to manage the wound exudate of highly exuding wounds. In a preferred embodiment, the antimicrobially active material comprising the hydrophilic polyurethane foam layer, forms preferably at least a part of the skin-contacting surface of a wound dressing.

To avoid leaching of the excess of exudate at the side borders of the wound covering due to a slow absorption of the exudate, which subsequently may cause maceration of the surrounding skin, it has been found suitable that the fluid absorption capacity after saturation of the antimicrobially active material of the invention is higher than 4 grams per gram material, preferably higher than 8 grams, preferably higher than 12 grams, and most preferably higher than 15 grams per gram dry material.

Because it is important that the wound dressing retains the wound exudate during removal of the wound dressing, it has been found suitable as well that the fluid absorption capacity after draining of the hydrophilic material of the wound covering is higher than 2, preferably higher than 6 grams, preferably higher than 10 grams, and most preferably higher than 13 grams per gram dry material.

To efficiently drain away the wound exudate, it has been found suitable that the wet out, this is the absorption velocity in seconds of a drop of 1 ml of water in the middle of the material, is lower than 500 s, preferably lower than 250 s, more preferably lower than 50 s and most preferably lower than 10 s.

The hydrophilic polyurethane foams can be produced according to the technology of polyurethane prepolymers. The prepolymer which is used in the method of the invention is preferably an isocyanate-capped polyether, such as a polyethyleneoxide or an ethyleneoxy/propyleneoxy copolymer. A particularly suitable prepolymer is described in GB-A-1 429 711 and in WO96/16099 or US6271277. Suitable prepolymers are commercially available under the name HYPOL® (Dow Chemical) or as Optipol (LMI).

The hydrophilic polyurethane foam has preferably an open-celled, very fine cell structure with an average cell diameter in the range of 100 to 800 microns, since such foams are found to be appropriate for low adherence wound dressings.

To prepare the antimicrobially active material of the invention, an antimicrobially active agent is covalently bonded to the foam. This antimicrobially active agent consists of a quaternary ammonium compound, having an apolar end group and a reactive group that can provide a covalent bond with the polymeric substrate. Suitable antimicrobially active agents are disclosed in US-A-4 631 297 (Dow Corning). The quaternary ammonium compound corresponds for example to one of the formulae

$R\text{-}R'\text{-}N^{+}R''R'''R''''X^{-}$ and

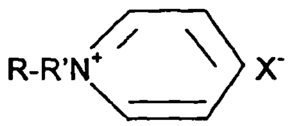

wherein in each formula,

R is a reactive group by means of which the quaternary ammonium compound is covalently bonded to the polyurethane foam;
R' is an alkylene group of 1 to 4 carbon atoms;
R'' and R''' are each independently selected from the group consisting of alkyl radicals of 1 to 20 carbon atoms, $-CH_2C_6H_5$, $-CH_2CH_2OH$, $-CH_2OH$, and $-(CH_2)_xNCH(O)R^v$, wherein x has a value of 2 to 10 and $R^v$ is a perfluoroalkyl radical having from 1 to 12 carbon atoms;
R'''' is selected from the group consisting of alkyl radicals of 6 to 20 carbon atoms, preferably of 8 to 20 carbon atoms and more preferably of 12 to 20 carbon atoms; $-CH_2C_6H_5$, and $-(CH_2)_xNCH(O)R^v$, wherein x has a value of 2 to 10 and $R^v$ is a perfluoroalkyl radical having from 1 to 12 carbon atoms when x>2 and 2 to 12 carbon atoms when x=2; and
X is an anion such as chloride, bromide, fluoride, iodide, acetate or tosylate.

The reactive group R may for example be a siloxane, an isocyanate, an alcohol, an epoxide or a vinyl or allyl monomer, substances that have the capability to form radicals and to polymerise under exposure of initiator molecules,

UV or other sources of radiation. Preferably, the reactive group R is a siloxane group, in particular a siloxane group of the formula

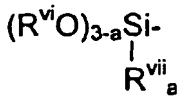

wherein

$R^{vi}$ is an alkyl radical of 1 to 4 carbon atoms or hydrogen;
a has a value of 0, 1 or 2; and
$R^{vii}$ is a methyl or ethyl radical.

More preferably, the quaternary ammonium compound is an organosilane, having the formula:

$$(CH_3O)_3Si(CH_2)_3N^+(CH_3)_2C_{18}H_{37}Cl^-$$

or

$$(CH_3O)_3Si(CH_2)_3N^+(C_{10}H_{21})_2(CH_3)Cl^-.$$

These antimicrobially active agents are for example available under the trade name AEGIS (Devan Chemicals).

The antimicrobially active quaternary ammonium compounds have an apolar end group which comprises preferably at least 6, more preferably at least 8 and most preferably at least 12 carbon atoms. The apolar end group is more preferably an apolar end chain comprising a carbon backbone of at least 6, preferably of at least 8 and more preferably of at least 12 carbon atoms and/or comprises an aromatic ring.

The antimicrobial activity of the quaternary ammonium compounds with the apolar end groups (i.e. of the resulting quaternary ammonium groups in the hydrophilic polyurethane foam) is based on the disruption of the cell membrane of the targeted micro-organisms. The disruption is caused by two mechanisms: the long apolar end group physically perforates the cell membrane, and the positively charged nitrogen component interferes with the ionic transport fluxes through the cell membranes. Due to these physical interactions, the targeted micro-organisms are not able to build up resistance against the antimicrobially active agent, which is a well-known effect and discussed issue in antibiotic treatment of infections.

The antimicrobial active agent can be incorporated in the polymeric structure after the formation thereof, by impregnation, spraying, dipping, brushing, padding or other foam finishing techniques. In order to achieve a more uniform distribution of the antimicrobially active agent, it is preferably incorporated in the polymeric substrate during the formation thereof, in particular during the foaming.

The objective of the wound dressing of the invention is to provide an antimicrobial wound dressing, showing a good absorbing capacity as well as a durable and enhanced antimicrobial effectiveness. It is also an objective to immobilise the active antibacterial component entirely in the substrate so that it does not leach into the environment and so that it is stable during the lifetime of the dressing.

US-A-4 631 297 (Dow Corning) discloses concentrations of the antimicrobial agent of between 1 and 5 parts per 100 parts polyol, the lowest concentrations causing however a substantially smaller reduction of the number of bacteria when measuring this reduction according to the shake flask method ASTM E 2149-01 (Aegis CTM0923 Dynamic Shake Flask). The tests performed in accordance with this method to achieve the results indicated hereinafter were performed with 1 g samples, 50 ml 0.3 mM $KH_2PO_4$ and with 1 x $10^5$ E. coli/ml ATCC 25922.

Surprisingly, it has been found according to the present invention that an important bactericidal activity was already obtained with smaller amounts of the antibacterial agent than described in US-A-4 631 297 when using a polyurethane foam substrate that is more hydrophilic than the hydrophobic polyurethane foam substrates disclosed in this US patent. The present inventor has incorporated for example the antimicrobial agent Aegis 5772 into a hydrophilic polyurethane foamduring the production thereof, starting from an isocyanate prepolymer, in amounts of respectively 0 parts (A), 1.15 parts (B), 0.76 parts (C), 0.37 parts (D) and 0.14 parts by weight (E) agent per 100 parts by weight dry foam substrate. The results in Table I show that concentrations as low as 0.37 parts agent per 100 parts dry foam substrate surprisingly still showed a 99.99% reduction of micro-organisms, in particular of bacteria, whereas a concentration of 0.14 parts agent per 100 parts dry foam substrate appeared to have no effect on E. coli. Further optimalisation may thus reduce the required amount of antimicrobial agent to a value between 0.37 and 0.14 parts per 100 parts dry foam.

Table I: Effect of the incorporation of different parts by weight of Aegis 5772 on the reduction of E. coli and Aspergillus niger and on the distribution of this antimicrobial agent in a hydrophilic polyurethane foam into which it is incorporated during the formation thereof.

| | Parts Aegis 5772/ 100 parts dry foam | Parts Lurol 8778/ 100 parts dry foam | Microbiological analysis (% reduction of Escherichia coli) | Fungal analysis Growth rating Aspergillus niger | Chemical analysis Homogeneous distribution of the antimicrobial agent (Bromophenol Blue colouring) |
|---|---|---|---|---|---|
| A | 0.0 | | 0.0 | 4 | Homogenous slightly blue |
| B | 1.15 | | 99.99 | 0 | Dark blue spots |
| C | 0.76 | | 99.99 | 0 | Dark blue spots |
| D | 0.37 | | 99.99 | 0 | Homogeneously blue |
| E | 0.14 | | 0.0 | 2 | Homogeneously blue |
| | | | | | |
| F | 1.15 | 0.3 | 99.99 | 0 | Homogeneously blue |
| G | 0.76 | 0.2 | 99.99 | 0 | Homogeneously blue |
| H | 0.76 | 0.5 | 99.99 | 0 | Homogeneously blue |

Moreover and even more surprisingly, these same low concentrations showed effective antifungal activity as well. The antifungal activity was tested according to the AATCC30 method, more particularly against the fungus Aspergillus niger. Down to concentrations of 0.37 parts antimicrobial agent by 100 weight parts of dry foam, no fungal growth was observed.

Surprisingly as well, the antimicrobially active materials made in accordance with the invention, still show highly hydrophilic properties, although the antimicrobial agent, which is hydrophobic, is not leaching. Table II shows the average values of the hydrophilic characteristics of the tested dressings.

Table II Physical characteristics of the foams indicated in Table I.

| | A | B | C | D | E | F | G | H |
|---|---|---|---|---|---|---|---|---|
| Cell size ($\mu$) | 100-220 | 300-400 | 300-400 | 300-400 | 300-400 | 300-400 | 300-400 | 250-350 |
| Wet out (s) | < 1 | 215 | 480 | <1 | <1 | 6 | 4 | 2 |
| Fluid capacity after saturation (g water/g dry material) | 22 | 16 | 16 | 20 | 23 | 15 | 16 | 18 |
| Fluid capacity after drain (g water/g dry material) | 21 | 8 | 8 | 13 | 22 | 8 | 8 | 15 |
| +% elongation dry | 271 | 178 | 187 | 203 | 255 | 176 | 176 | 238 |

In comparison with foam A (blank foam), foam samples B and C show a significantly increased wet out, as well as a decreased fluid capacity after saturation and a decreased fluid capacity after drain. This proves that the antimicrobial

agent renders the foam more hydrophobic. Foams D and E show similar absorption properties as the reference material without antimicrobial agent.

It was also found that addition of the antimicrobial agent may reduce the mechanical properties of the foam as shown in Table II. Surprisingly, this negative effect could be eliminated by reducing the concentration of the antimicrobial agent, as shown for Foam D, or by adding a hydrophilising additive as shown for foam H. In this case a polyether silicone derivative was used, known under the trade name Lurol 8728 (Devan Chemicals). Concentrations of 0.3 parts (F), 0.2 parts (G), and 0.5 parts by weight (H) Lurol 8728 per 100 parts by weight dry foam substrate were used.

In view of the hydrophobic character of the quaternary ammonium compound, this compound is preferably bonded to the hydrophilic polyurethane foam in an amount of less than 1 part by weight, preferably less than 0.80 parts by weight, more preferably less than 0.60 parts by weight, and most preferably less than 0.40 parts by weight per 100 parts by dry weight of said material. On the other hand, the quaternary ammonium compound is preferably covalently bonded to the hydrophilic polyurethane foam in an amount of at least 0.15 parts by weight, preferably at least 0.20 parts by weight, and more preferably at least 0.25 parts by weight per 100 parts by dry weight of said material in view of the desired antimicrobial activity which is to be achieved.

Without limiting the invention to any specific theory it is assumed by the present inventor that the micro-organisms are drained away from the wound site, together with the wound exudate. After contacting the wound dressing surface they will die out and microorganism residues will be removed from the wound site with exchange of the dressing.

In addition to the covalently bonded antimicrobial agent, the wound dressing of the invention may also include topical medicaments and other leachable antiseptics, as well as other therapeutically useful additives such as polypeptides, growth factors and enzymes. The hydrophilic polyurethane foam may further comprise all kinds of additives, including additives which improve the hydrophilic nature of the foam or increase the fluid absorption capabilities.

The wound dressing or antimicrobially active flexible polyurethane foam may show whether or not adhesive properties. In the last case it will then typically be secured on the desired wound site using conventional means such as a cover dressing.

In a preferred embodiment of the invention, the dressing comprises a skin-contacting surface comprising an area showing a skin friendly adhesive. Such a dressing may also suitably comprise a water-impervious backing layer, film or membrane whereto a skin-friendly adhesive is applied, to which the absorbing, antimicrobial polyurethane foam substrate is applied, forming an island dressing. The water impervious layer, film or membrane may be of any suitable material known in the preparation of wound dressings as a non-woven layer, a foam, a polyurethane, polyethylene, polyester or polyamide film. The skin friendly adhesive may exist of adhesive hydrocolloids or medical grade acrylate based adhesives.

The adhesive applied to the polyurethane foam is preferably water-based. According to the invention it has been found that such a water-based adhesive adheres quite well to the foam and is in particular not released when the hydrophilic polyurethane foam swells as a result of the water absorption. This advantage can possibly be explained by the physical entanglement of the adhesive in the surface cells of the foam which can be achieved by means of a water-based adhesive. This effect is in particular more pronounced compared to the conventional transfer coating of adhesive layers or the application of hot melt glues. Compared to the solvent based adhesives, a water-based adhesive can be applied more easily without causing any problem of volatile solvents.

To make a self-adherent wound dressing, commonly an extra processing step of transfer coating of an adhesive layer to the foam substrate is carried out. Water-based adhesives are not used in practice on hydrophilic polyurethane foams, since this implies an additional drying step. However, the present inventor has found that such an additional drying step is not required when the hydrophilic polyurethane foam is produced from a curable composition containing an excess of water and when the water-based adhesive is applied before this excess of water is entirely removed by the drying step which is required to produce the hydrophilic polyurethane foam. In this way, the water of the water-based adhesive is evaporated at the same time as the excess of water in the produced polyurethane foam. In a preferred embodiment, the curable composition is a foamable composition. The curable composition may comprise an isocyanate component, in particular an isocyanate prepolymer, mixed with an aqueous component. The water in the aqueous component reacts with the free isocyanate groups to produce $CO_2$ as blowing agent and provides for a crosslinking between the prepolymer molecules. Some of the water will thus react during the foam formation. However, since the curable composition contains an excess of water, the obtained foam has to be dried afterwards to remove this excess.

Preferably a water based, medical grade acrylate based adhesive is applied during formation of the hydrophilic polyurethane foam substrate, that may comprise a covalently bonded antimicrobial agent. Surprisingly to all other methods this can be done in a 1 step process, i.e. without requiring additional steps after the production of the foam. After the foam is blown up, a medical grade, water based adhesive is laid down on the curing foam. Subsequently the polymeric substrate and the adhesive layer is dried. A suitable water based adhesive is Aqualine PSA 1623 (Collano). All known methods, such as but not limited to reverse roll coating, kiss coating, flat die system or rotary screen printing can be used. Most preferably a dot screen printing is applied. All kind of patterns can be applied, but preferably a dot with a diameter between 0.4 and 4 mm, preferably between 1 and 1.5 mm, with a distance between the dots varying between

0.3 and 8 mm, and preferably between 0.5 and 2.5 mm. This guarantees that the hydrophilic properties of the polymeric foam substrate are maintained (see table III). The total area of the polymeric foam substrate surface can be covered with adhesive. However, more preferably only the side borders, aimed to cover healthy skin, are coated with adhesive dots. To obtain sufficient adherence strength, that allows normal movement of the body parts, but does not cause trauma or pain at removal of the dressing, adhesive amounts between 25 $g/m^2$ and 300 $g/m^2$ are applied, preferably between 50 $g/m^2$ and 250 $g/m^2$, and more preferably between 100 $g/m^2$ and 200 $g/m^2$ (measured over the entire foam surface covered by a continuous adhesive coating or by distinct adhesive dots).

Table III: Hydrophilic properties of a hydrophilic polyurethane foam having applied thereon dots of an adhesive.

| | Foam without adhesive | Foam with screen print dot application of adhesive Aqualine PSA 1623 |
|---|---|---|
| Wet out (s) | 4 | 10 |
| Fluid capacity after 3 min (g water/g foam) | 16.3 | 21.4 |
| Fluid capacity after 30 min (=saturated) (g water/g foam) | 21.4 | 21.4 |

After the drying process of the foam-adhesive compound, preferably by microwave energy, the surface at the side of the adhesive is covered by a release layer. Subsequently, the self-adherent foam can be converted into its final dressing dimensions.

The hydrophilic foam substrate can be produced by a process comprising the steps of:

1) intensively premixing a reactive antimicrobial agent (if required) with a component stream A which is preferably an aqueous stream, comprising surfactants and/or any other active fillers;
2) mixing the A stream with the other product stream or streams, preferably a stream with one or more isocyanate prepolymers;
3) transforming the mixture into a thin layer having a predetermined thickness;
4) if desired, applying a water based adhesive;
5) drying and curing, preferably with microwave technology, the thin layer; and
6) converting the thin layer to the final dressing design.

The hydrophilic foam substrate may also be produced directly from a separate polyol and isocyanate stream, either or not in combination with the aqueous stream, and additionally with catalysts, stabilisers or colours streams.

Instead of mixing the reactive antimicrobial agent first with component stream A, it can also be supplied in a separate stream which is mixed with the other streams to start the foaming reaction. If the hydrophilic polyurethane should have no antimicrobial properties, the antimicrobial agent can be omitted.

The dressing of the invention has mainly been described with reference to wound dressings, but it will be evident for the skilled in the art that the invention is not limited to wound dressings. Thus a medical dressing of the invention may be used in wound care, incontinence care, ostomy care and is even not limited to these applications. It could be also used as pad or swob in skin care and cosmetic care in general.

**Example**

Materials

Hypol 2002, Dow
Optipol, LMI
Pluronic 6200 PO-PE block copolymer, BASF
Brij 58, Uniquema
Ion exchanged water from internal laboratory supply
Bromophenolblue
Aegis 5772
Lurol 8728

Aqualine PSA 1623

Methods

1. WET-OUT: DRY

■ Size : rectangular sample.

- Requirements: pipet of 1 ml, demineralized water

■ Method : Bring a drop of 1 ml on a the horizontal surface of the sample and measure the time (in seconds) that the drop of water needs to penetrate totally in the foam.
■ Result : Wet-out dry in seconds

2. FLUID CAPACITY AFTER SATURATION

■ EN13726-1
■ Size : Two rectangular samples (63 x 63 x d mm)
■ Requirements :

- Balance
- Recipient filled with water (water temperature = 37 ± 0.5°C)

■ Method :

- weigh the sample dry ($G_0$)
- immerse the sample in the water during 30 min
- take the sample horizontally out of the water so that no water drops out (minimum water loss)
- Weigh the sample immediately ($G_1$)

■ Calculation :

$$\text{Ratio g water / g product} = \frac{(G_1 - G_0)}{G_0}$$

3. FLUID CAPACITY AFTER DRAIN

- EN 13726-1

  ■ Size : Two rectangular samples (63 x 63 x d mm)
  ■ Requirements :

  - Balance
  - Recipient filled with water (water temperature = 37 ± 0.5°C)

  ■ Method :

  - weigh the sample dry ( $G_0$).
  - immerse the sample in the water during 30 min
  - Take the sample out of the water, taking it at one corner, let drop out the excess of not bonded water during

30 seconds
- weigh the sample (G).

■ Calculation :

$$\text{Ratio g water / g product} = \frac{(G_1 - G_0)}{G_0}$$

4. DYNAMIC SHAKE FLASK METHOD

ASTM E2149-01

Description of the method see Standard Test "C" described on p. 19 to 21 of EP-A-0 155 155, which is incorporated herein by way of reference.

5. BROMOPHENOL BLUE TEST

Description of the method see Standard Test "B" described on p. 17 to 19 of EP-A-0 155 155, which is incorporated herein by way of reference.

6. FUNGAL GROWTH TEST

AATCC Test Method 30-1999

Example

A polyurethane foam sheet was produced by mixing Hypol 2002 (100 g), water (85g), Pluronic 6200 (1g) and Aegis 5772 (1.5, 0.76, 0.37 and 0.14 g respectively), by first mixing the three last components and then adding this mixture to the prepolymer during mixing.

The mixture was poured down on silicone paper and transformed with a doctor roll and guiding bars to a thin sheet of desired thickness, more particularly so that after foaming a foam layer having a thickness of about 5 mm is achieved. When the material was foamed, it was dried and cured using microwave technology or a ventilated dry air oven.

Alternatively, three separate streams of respectively the prepolymer Hypol 2002, the aqueous phase of solved surfactant Pluronic 6200 and the antimicrobial agent Aegis 5772 (TMS, 3-(trimethoxysilyl)-propyloctadecyldimethylammonium chloride) were injected in the dynamix mixer, pouring down this mixture on silicone paper.

The presence of the active agent was demonstrated and qualitatively determined with the bromophenol blue test. In the Agar diffusion test (SN195920), no zone of inhibition was observed, demonstrating that the active agent was not leaching out of the foam.

Antimicrobial tests (shake flask method) showed a significant reduction of bacterial contamination.

Similar results were obtained when repeating this example with 3-(trimethoxysilyl)-propylhexyldimethylammonium chloride and with 3-(trimethoxysilyl)-propyldodecyldimethylammonium chloride.

## Claims

**1.** An antimicrobially active flexible hydrophilic material comprising a hydrophilic polyurethane foam, **characterised in that** said material comprises further at least one antimicrobially active quaternary ammonium compound which has an apolar end group and which is covalently bonded to the hydrophilic polyurethane foam in such an amount that the material is antimicrobially active and has:

- a fluid absorption capacity after saturation , of higher than 4 g per gram material, preferably higher than 8 g, more preferably higher than 12 g, and most preferably higher than 15 g per gram material;
- a fluid absorption capacity after drain, of higher than 2 g, preferably higher than 6 g, more preferably higher than 10 g, and most preferably higher than 13 g per gram material; and
- a wet out, lower than 500 s, preferably lower than 250 s, more preferably lower than 50 s and most preferably lower than 10 s.

**2.** A material according to claim 1, **characterised in that** said apolar end group comprises at least 6, preferably at least 8 and more preferably at least-12 carbon atoms.

**3.** A material according to claim 1 or 2, **characterised in that** said apolar end group is an apolar end chain comprising a carbon backbone of at least 6, preferably of at least 8 and more preferably of at least 12 carbon atoms and/or comprises an aromatic ring.

**4.** A material according to any one of the claims 1 to 3, **characterised in that** said quaternary ammonium compound comprises a reactive group by means of which it is covalently bonded to the polyurethane foam, the reactive group being in particular a siloxane, an isocyanate, an alcohol, an epoxide, or a vinyl or allyl monomer.

**5.** A material according to any one of the claims 1 to 4, **characterised in that** said quaternary ammonium compound is covalently bonded to the hydrophilic polyurethane foam in an amount of less than 1 part by weight, preferably less than 0.80 parts by weight, more preferably less than 0.60 parts by weight, and most preferably less than 0.40 parts by weight per 100 parts by dry weight of said material.

**6.** A material according to any one of the claims 1 to 5, **characterised in that** said quaternary ammonium compound is covalently bonded to the hydrophilic polyurethane foam in an amount of at least 0.15 parts by weight, preferably at least 0.20 parts by weight, and more preferably at least 0.25 parts by weight per 100 parts by dry weight of said material.

**7.** A material according to any one of the claims 1 to 6, **characterised in that** a hydrophilizing agent is incorporated in the hydrophilic polyurethane foam to improve the hydrophilic and mechanical properties thereof.

**8.** A material according to any one of the claims 1 to 7, **characterised in that** said quaternary ammonium compound has a formula selected from the group consisting of

$$R\text{-}R'\text{-}N^{+}R''R'''R''''X^{-}$$

and

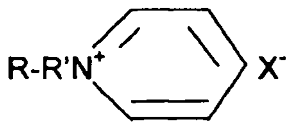

wherein in each formula,

R is a reactive group by means of which the quaternary ammonium compound is covalently bonded to the polyurethane foam;
R' is an alkylene group of 1 to 4 carbon atoms;
R'' and R''' are each independently selected from the group consisting of alkyl radicals of 1 to 20 carbon atoms, $-CH_2C_6H_5$, $-CH_2CH_2OH$, $-CH_2OH$, and $-(CH_2)_xNCH(O)R^v$, wherein x has a value of 2 to 10 and $R^v$ is a perfluoroalkyl radical having from 1 to 12 carbon atoms;
R'''' is selected from the group consisting of alkyl radicals of 6 to 20 carbon atoms, preferably of 8 to 20 carbon atoms and more preferably of 12 to 20 carbon atoms; $-CH_2C_6H_5$, and $-(CH_2)_xNCH(O)R^v$, wherein x has a value of 2 to 10 and $R^v$ is a perfluoroalkyl radical having from 1 to 12 carbon atoms when x>2 and 2 to 12 carbon atoms when x=2; and;
X is an anion such as chloride, bromide, fluoride, iodide, acetate or tosylate.

**9.** A material according to claim 8, **characterised in that** R is a reactive siloxane group, in particular a siloxane group of the formula

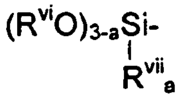

wherein

$R^{vi}$ is an alkyl radical of 1 to 4 carbon atoms or hydrogen;
a has a value of 0, 1 or 2; and
$R^{vii}$ is a methyl or ethyl radical.

**10.** A material according to claim 9, **characterised in that** said quaternary ammonium compound has the formula

$$(CH_3O)_3Si(CH_2)_3N^+(CH_3)_2C_{18}H_{37}Cl^-.$$

**11.** A material according to claim 9, **characterised in that** said quaternary ammonium compound has the formula

$$(CH_3O)_3Si(CH_2)_3N^+(C_{10}H_{21})_2(CH_3)Cl^-.$$

**12.** A wound dressing comprising an antimicrobially active polymeric material according to any one of the previous claims.

**13.** A wound dressing according to claim 12, **characterised in that** the hydrophilic polyurethane foam comprises at least one leachable antiseptic, topical medicament and/or another therapeutically useful additive such as a polypeptide, a growth factor or an enzyme.

**14.** A wound dressing according to claim 12 or 13, **characterised in that** the hydrophilic polyurethane foam comprises an additive which improves the hydrophilic nature of the foam and/or an additive which increases the fluid absorption capabilities.

**15.** A wound dressing according to any one of the claims 12 to 14, **characterised in that** it further comprises a water-impervious backing layer, film or membrane whereto an adhesive is applied, to which the antimicrobially active polymeric material is applied, forming an island dressing.

**16.** A wound dressing according to any one of the claims 12 to 15, **characterised in that** the hydrophilic polyurethane foam is provided with an adhesive at the wound covering surface to be self adherent, the adhesive being applied on the surface of the polyurethane foam.

**17.** A wound dressing according to claim 16, **characterised in that** said adhesive is a water based polyacrylate based adhesive.

**18.** A wound dressing according to claim 16 or 17, **characterised in that** said adhesive is applied by dot screen printing.

**19.** A wound dressing according to any one of the claims 16 to 18, **characterised in that** said adhesive is a water based polyacrylate based adhesive, applied in amounts of between 25 $g/m^2$ and 300 $g/m^2$, preferably between 50 $g/m^2$ and 250 $g/m^2$, and more preferably between 100 $g/m^2$ and 200 $g/m^2$.

## Patentansprüche

**1.** Ein antimikrobiell aktives flexibles hydrophiles Material, welches einen hydrophilen Polyurethanschaum umfasst, **dadurch gekennzeichnet, dass** das erwähnte Material ferner zumindest eine antimikrobiell aktive quaternäre Ammoniumverbindung enthält, die eine apolare Endgruppe hat und die in einer solchen Menge kovalent mit dem hydrophilen Polyurethanschaum verbunden ist, dass das Material antimikrobiell aktiv ist und Folgendes hat:

- eine Flüssigkeitsabsorptionsfähigkeit nach Saturation von mehr als 4 g pro Gramm Material, vorzugsweise mehr als 8 g, besser mehr als 12 g und am besten mehr als 15 g pro Gramm Material;
- eine Flüssigkeitsabsorptionsfähigkeit nach Ablauf von mehr als 2 g, vorzugsweise mehr als 6 g, besser mehr als 10 g und am besten mehr als 13 g pro Gramm Material; und

- eine Vernetzung unter 500 s, vorzugsweise unter 250 s, besser unter 50 s und am besten unter 10 s.

2. Ein Material nach Anspruch 1, **dadurch gekennzeichnet, dass** die erwähnte apolare Endgruppe zumindest 6, vorzugsweise zumindest 8 und besser zumindest 12 Kohlenstoffatome umfasst.

3. Ein Material nach Anspruch 1 oder 2, **dadurch gekennzeichnet, dass** die erwähnte apolare Endgruppe eine apolare Endkette mit einer Kohlenstoff-Hauptkette aus zumindest 6, vorzugsweise aus zumindest 8 und besser aus zumindest 12 Kohlenstoffatomen umfasst und/oder einen aromatischen Ring umfasst.

4. Ein Material nach jedem der Ansprüche 1 bis 3, **dadurch gekennzeichnet, dass** die erwähnte quaternäre Ammoniumverbindung eine reaktive Gruppe umfasst, mittels der sie kovalent mit dem Polyurethanschaum verbunden ist, wobei die reaktive Gruppe insbesondere ein Siloxan, ein Isocyanat, ein Alkohol, ein Epoxid oder ein Vinyl- oder Allylmonomer ist.

5. Ein Material nach jedem der Ansprüche 1 bis 4, **dadurch gekennzeichnet, dass** die erwähnte quaternäre Ammoniumverbindung kovalent mit dem hydrophilen Polyurethanschaum in einer Menge von weniger als 1 Gewichtsteil, vorzugsweise weniger als 0,80 Gewichtsteile, besser weniger als 0,60 Gewichtsteile und am besten weniger als 0,40 Gewichtsteile pro 100 Trockengewichtsteile des erwähnten Materials verbunden ist.

6. Ein Material nach jedem der Ansprüche 1 bis 5, **dadurch gekennzeichnet, dass** die erwähnte quaternäre Ammoniumverbindung kovalent mit dem hydrophilen Polyurethanschaum in einer Menge von zumindest 0,15 Gewichtsteilen, vorzugsweise zumindest 0,20 Gewichtsteile und besser zumindest 0,25 Gewichtsteile pro 100 Trockengewichtsteile des erwähnten Materials verbunden ist.

7. Ein Material nach jedem der Ansprüche 1 bis 6, **dadurch gekennzeichnet, dass** ein Hydrophilisator in den hydrophilen Polyurethanschaum integriert ist, um dessen hydrophile und mechanische Eigenschaften zu verbessern.

8. Ein Material nach jedem der Ansprüche 1 bis 7, **dadurch gekennzeichnet, dass** die erwähnte quaternäre Ammoniumverbindung eine Formel hat, ausgewählt aus der Gruppe bestehend aus

$$R\text{-}R'\text{-}N^+R''R'''R''''X^-$$

und

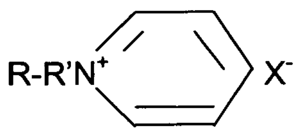

wobei in jeder Formel

R eine reaktive Gruppe ist, mittels der die quaternäre Ammoniumverbindung kovalent mit dem Polyurethanschaum verbunden ist;
R' eine Alkylengruppe aus 1 bis 4 Kohlenstoffatomen ist;
R'' und R''' jeweils unabhängig aus der Gruppe bestehend aus Alkylradikalen aus 1 bis 20 Kohlenstoffatomen, $-CH_2C_6H_5$, $-CH_2CH_2OH$, $-CH_2OH$, und $-(CH_2)_xNCH(O)R^v$ ausgewählt werden, wobei x einen Wert von 2 bis 10 hat und $R^v$ ein Perfluoralkylradikal mit 1 bis 12 Kohlenstoffatomen ist;
R'''' aus der Gruppe bestehend aus Alkylradikalen aus 6 bis 20 Kohlenstoffatomen, vorzugsweise aus 8 bis 20 Kohlenstoffatomen und besser aus 12 bis 20 Kohlenstoffatomen; $-CH_2C_6H_5$, und $-(CH_2)_xNCH(O)R^v$ ausgewählt wird, wobei x einen Wert von 2 bis 10 hat und $R^v$ ein Perfluoralkylradikal mit 1 bis 12 Kohlenstoffatomen ist, wenn x>2 und mit 2 bis 12 Kohlenstoffatomen, wenn x=2; und;
X ein Anion wie Chlorid, Bromid, Fluorid, Iodid, Acetat oder Tosylat ist.

9. Ein Material nach Anspruch 8, **dadurch gekennzeichnet, dass** R eine reaktive Siloxangruppe ist, insbesondere eine Siloxangruppe der Formel

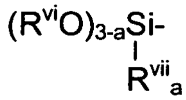

wobei

$R^{vi}$ ein Alkylradikal aus 1 bis 4 Kohlenstoffatomen oder Wasserstoff ist;
a einen Wert von 0, 1 oder 2 hat; und
$R^{vii}$ ein Methyl- oder Ethylradikal ist.

**10.** Ein Material nach Anspruch 9, **dadurch gekennzeichnet dass** die erwähnte quaternäre Ammoniumverbindung die Formel $(CH_3O)Si(CH_2)_3N^+(CH_3)_2C_{18}H_{37}Cl^-$ hat.

**11.** Ein Material nach Anspruch 9, **dadurch gekennzeichnet dass** die erwähnte quaternäre Ammoniumverbindung die Formel $(CH_3O)_3Si(CH_2)_3N^+(C_{10}H_{21})_2CH_3)Cl^-$ hat.

**12.** Ein Wundverband bestehend aus einem antimikrobiell aktiven polymeren Material nach jedem der vorigen Ansprüche.

**13.** Ein Wundverband nach Anspruch 12, **dadurch gekennzeichnet, dass** der hydrophile Polyurethanschaum zumindest ein auswaschbares Antiseptikum, topisches Arzneimittel und/oder einen anderen therapeutisch nützlichen Zusatz wie ein Polypeptid, einen Wachstumsfaktor oder ein Enzym umfasst.

**14.** Ein Wundverband nach Anspruch 12 oder 13, **dadurch gekennzeichnet, dass** der hydrophile Polyurethanschaum einen Zusatz umfasst, welcher die hydrophile Art des Schaums verbessert, und/oder einen Zusatz, welcher die Flüssigkeitsabsorptionsfähigkeiten erhöht.

**15.** Ein Wundverband nach jedem der Ansprüche 12 bis 14, **dadurch gekennzeichnet, dass** er ferner eine wasserundurchlässige Rückenschicht, Folie oder Membran umfasst, auf die ein Kleber aufgetragen ist, an dem das antimikrobiell aktive polymere Material angebracht ist, wodurch ein Wundverband mit Insel entsteht.

**16.** Ein Wundverband nach jedem der Ansprüche 12 bis 15, **dadurch gekennzeichnet, dass** der hydrophile Polyurethanschaum mit einem Kleber an der die Wunde bedeckenden Oberfläche ausgestattet ist, um selbst klebend zu sein, wobei der Kleber auf die Oberfläche des Polyurethanschaums aufgetragen ist.

**17.** Ein Wundverband nach Anspruch 16, **dadurch gekennzeichnet, dass** der erwähnte Kleber ein Polyacrylatkleber auf Wasserbasis ist.

**18.** Ein Wundverband nach Anspruch 16 oder 17, **dadurch gekennzeichnet, dass** der erwähnte Kleber durch Punktrasterdruck angebracht wird.

**19.** Ein Wundverband nach jedem der Ansprüche 16 bis 18, **dadurch gekennzeichnet, dass** der erwähnte Kleber ein Polyacrylatkleber auf Wasserbasis ist, aufgetragen in Mengen von zwischen 25 $g/m^2$ und 300 $g/m^2$, vorzugsweise zwischen 50 $g/m^2$ und 250 $g/m^2$ und besser zwischen 100 $g/m^2$ und 200 $g/m^2$.

## Revendications

**1.** Matériau hydrophile flexible à activité antimicrobienne comprenant une mousse de polyuréthanne hydrophile, **caractérisé en ce que** ledit matériau comprend en outre au moins un composé d'ammonium quaternaire à activité antimicrobienne qui a un groupe terminal apolaire et qui est lié de manière covalente avec la mousse de polyuréthanne dans une quantité telle que le matériau a une activité antimicrobienne et a :

- une capacité d'absorption de fluide après saturation supérieure à 4 g par gramme de matériau, de préférence supérieure à 8 g, mieux encore supérieure à 12 g, et idéalement supérieure à 15 g par gramme de matériau,
- une capacité d'absorption de fluide après drainage supérieure à 2 g, de préférence supérieure à 6 g, mieux encore supérieure à 10 g, et idéalement supérieure à 13 g par gramme de matériau, et

- un « wet out » (vitesse d'absorption en secondes d'une goutte de 1 ml d'eau au centre du matériau) inférieur à 500 s, de préférence inférieur à 250 s, mieux encore inférieur à 50 s et idéalement inférieur à 10 s.

**2.** Matériau selon la revendication 1, **caractérisé en ce que** ledit groupe terminal apolaire comprend au moins 6, de préférence au moins 8 et mieux encore au moins 12 atomes de carbone.

**3.** Matériau selon la revendication 1 ou 2, **caractérisé en ce que** ledit groupe terminal apolaire est une chaîne terminale apolaire comprenant un squelette carboné d'au moins 6, de préférence d'au moins 8 et mieux encore d'au moins 12 atomes de carbone et / ou comprend un cycle aromatique.

**4.** Matériau selon l'une quelconque des revendications 1 à 3, **caractérisé en ce que** ledit composé d'ammonium quaternaire comprend un groupe réactif au moyen duquel il est lié de manière covalente avec la mousse de polyuréthanne, le groupe réactif étant en particulier un siloxane, un isocyanate, un alcool, un époxyde, ou un monomère de vinyle ou d'allyle.

**5.** Matériau selon l'une quelconque des revendications 1 à 4, **caractérisé en ce que** ledit composé d'ammonium quaternaire est lié de manière covalente avec la mousse de polyuréthanne dans une quantité de moins de 1 part en poids, de préférence de moins de 0,80 part en poids, mieux encore de moins de 0,60 part en poids, et idéalement de moins de 0,40 part en poids par 100 parts en poids sec dudit matériau.

**6.** Matériau selon l'une quelconque des revendications 1 à 5, **caractérisé en ce que** ledit composé d'ammonium quaternaire est lié de manière covalente avec la mousse de polyuréthanne dans une quantité d'au moins 0,15 part en poids, de préférence d'au moins 0,20 part en poids, et mieux encore d'au moins 0,25 part en poids par 100 parts en poids sec dudit matériau.

**7.** Matériau selon l'une quelconque des revendications 1 à 6, **caractérisé en ce qu'**un agent hydrophilisant est incorporé dans la mousse de polyuréthanne hydrophile pour améliorer ses propriétés hydrophiles et mécaniques.

**8.** Matériau selon l'une quelconque des revendications 1 à 7, **caractérisé en ce que** ledit composé d'ammonium quaternaire a une formule choisie dans le groupe consistant en

$$R\text{-}R'\text{-}N^+R''R'''R''''X^-$$

et

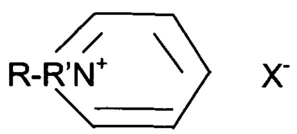

où dans chaque formule,

R est un groupe réactif au moyen duquel le composé d'ammonium quaternaire est lié de manière covalente avec la mousse de polyuréthanne;
R' est un groupe alkylène de 1 à 4 atomes de carbone;
R'' et R''' sont choisis indépendamment dans le groupe consistant en radicaux alkyle de 1 à 20 atomes de carbones, $-CH_2C_6H_5$, $-CH_2CH_2OH$, $-CH_2OH$, et $-(CH_2)_xNCH(O)R^v$ où x a une valeur de 2 à 10 et $R^v$ est un radical perfluoroalkyle ayant de 1 à 12 atomes de carbone;
R'''' est choisi dans le groupe consistant en radicaux alkyle de 6 à 20 atomes de carbone, de préférence de 8 à 20 atomes de carbone et mieux encore de 12 à 20 atomes de carbone; $-CH_2C_6H_5$, et $-(CH_2)_xNCH(O)R^v$, où x a une valeur de 2 à 10 et $R^v$ est un radical perfluoroalkyle ayant de 1 à 12 atomes de carbone quand $x > 2$ et de 2 à 12 atomes de carbone quand $x = 2$; et
X est un anion tel que du chlorure, du bromure, de l'iodure, de l'acétate ou du tosylate.

**9.** Matériau selon la revendication 8, **caractérisé en ce que** R est un groupe siloxane réactif, en particulier un groupe siloxane de la formule

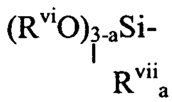

où

$R^{vi}$ est un radical alkyle de 1 à 4 atomes de carbone ou de l'hydrogène,
a a une valeur de 0, 1 ou 2; et
$R^{vii}$ est un radical méthyle or éthyle.

**10.** Matériau selon la revendication 9, **caractérisé en ce que** ledit composé d'ammonium quaternaire a la formule

$$(CH_3O)_3Si(CH_2)_3N^+(CH_3)_2C_{18}H_{37}Cl^-.$$

**11.** Matériau selon la revendication 9, **caractérisé en ce que** ledit composé d'ammonium quaternaire a la formule

$$(CH_3O)_3Si(CH_2)_3N^+(C_{10}H_{21})_2(CH_3)Cl^-.$$

**12.** Pansement comprenant un matériau polymère à activité antimicrobienne selon l'une quelconque des revendications précédentes.

**13.** Pansement selon la revendication 12, **caractérisé en ce que** la mousse de polyuréthanne hydrophile comprend au moins un médicament topique antiseptique lixiviable et / ou un autre additif à usage thérapeutique tel qu'un polypeptide, un facteur de croissance ou une enzyme.

**14.** Pansement selon la revendication 12 ou 13, **caractérisé en ce que** la mousse de polyuréthanne hydrophile comprend un additif qui améliore la nature hydrophile de la mousse et / ou un additif qui accroît les capacités d'absorption de fluide.

**15.** Pansement selon l'une quelconque des revendications 12 à 14, **caractérisé en ce qu'**il comprend en outre une couche support, un film ou une membrane imperméable à l'eau sur laquelle est appliqué un adhésif auquel est appliqué le matériau polymère à activité antimicrobienne formant un pansement à îlot central.

**16.** Pansement selon l'une quelconque des revendications 12 à 15, **caractérisé en ce que** la mousse de polyuréthanne hydrophile est pourvue d'un adhésif au niveau de la surface de couverture de la plaie pour être autoadhésive, l'adhésif étant appliqué sur la surface de la mousse de polyuréthanne.

**17.** Pansement selon la revendication 16, **caractérisé en ce que** ledit adhésif est un adhésif à base de polyacrylate à base aqueuse.

**18.** Pansement selon la revendication 16 ou 17, **caractérisé en ce que** ledit adhésif est appliqué par sérigraphie par points.

**19.** Pansement selon l'une quelconque des revendications 16 à 18, **caractérisé en ce que** ledit adhésif est un adhésif à base de polyacrylate à base aqueuse, appliqué en quantités comprises entre 25 g / m$^2$ et 300 g / m$^2$, de préférence entre 50 g / m$^2$ et 250 g / m$^2$, et mieux encore entre 100 g / m$^2$ et 200 g / m$^2$.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20030088202 A
- WO 9913923 A
- WO 02062403 A
- US 4631297 A
- US 20020177828 A
- US 5045322 A
- GB 1429711 A
- WO 9616099 A
- US 6271277 B
- EP 0155155 A